Europäisches Patentamt

European Patent Office

Office européen des brevets

(11) Publication number: **0 397 362 B1**

(12) **EUROPEAN PATENT SPECIFICATION**

(45) Date of publication of patent specification: **21.06.95**   (51) Int. Cl.[6]: **G01N 33/48**

(21) Application number: **90304657.1**

(22) Date of filing: **30.04.90**

(54) **Electrochemical detection of growth of micro-organisms.**

(30) Priority: **08.05.89 GB 8910539**
        **18.09.89 GB 8921068**

(43) Date of publication of application:
    **14.11.90 Bulletin 90/46**

(45) Publication of the grant of the patent:
    **21.06.95 Bulletin 95/25**

(84) Designated Contracting States:
    **AT BE CH DE DK ES FR GB GR IT LI LU NL SE**

(56) References cited:
    **EP-A- 0 036 274**
    **GB-A- 1 433 887**
    **GB-A- 2 000 805**
    **GB-A- 2 177 801**
    **US-A- 4 009 078**

(73) Proprietor: **CarnaudMetalbox plc**
**Downsview Road**
**Wantage, Oxon OX12 9BP (GB)**

(72) Inventor: **Ackland, Martin Robert**
**Cedar Cottage,**
**Ginge Road,**
**West Hendred**
**Wantage,**
**Oxfordshire OX12 8RT (GB)**

Inventor: **Blundell, John Keith**
**28 Woodhill Drive**
**Grove,**
**Wantage,**
**Oxfordshire OX12 0DF (GB)**
Inventor: **Hedges, William Michael**
**28 Falcon View,**
**Badger Farm**
**Winchester,**
**Hampshire SO22 4EP (GB)**
Inventor: **Walpole, James Frederick**
**"Woodleigh"**
**Gainfield Buckland**
**Faringdon,**
**Oxfordshire SN7 8OO (GB)**

(74) Representative: **Sawers, Lawrence Peter et al**
**PAGE, WHITE & FARRER**
**54 Doughty Street**
**London WC1N 2LS (GB)**

EP 0 397 362 B1

**Description**

This invention relates to the electrochemical detection of growth of micro-organisms.

Apparatus and methods are known for the detection of growth of micro-organisms using a sterile nutrient medium in which are placed two electrodes (anode and cathode). The growth of a micro-organism placed in the nutrient medium can be detected by measuring the changing potential between the electrodes. It has been observed that when growth of the micro-organisms reaches a particular stage the potential shows a marked change, e.g. a steep fall.

In our British Patent Specification No 2142433 the Applicants have described an apparatus by which the growth of micro-organisms in a plurality of samples can be monitored electrochemically, by monitoring the potential difference developed galvanically between two electrodes of different metals, e.g. gold and aluminium, in contact with the samples.

Further, in the Applicants' European Patent Application Publication No EP-A-0313411 there is described a method of detecting micro-organisms in a cell in which an electrical D.C. input is applied across the two electrodes while maintaining constant current or constant potential conditions in the cell, and monitoring either voltage or current respectively in the cell against time. While these and other similar systems have been used successfully for detection of micro-organisms, the underlying mechanism giving rise to monitorable electrical changes has not hitherto been fully understood.

It has been discovered by the present inventors that the underlying mechanism involves the reduction of free dissolved oxygen at the cathode in the transfer of electrical charge between the electrodes. Whilst for aerobic samples using gold and aluminium electrodes there is a measurable potential of the order of 400-500mV in the absence of micro-organisms, falling to 200-300mV with the growth of micro-organisms and consumption by them of the dissolved oxygen, for anaerobic cells the initial measurable potential is extremely low or even zero because of the absence of dissolved oxygen. With this condition it is on the face of it impossible to use the known electrochemical detection techniques for anaerobic organisms.

The present invention has been developed to enable the above-described electrochemical detection techniques to be used for anaerobic organisms.

Other techniques are known for the detection of micro-organisms. US-A-4009078 discloses that electrostatic charges are associated with all cell growth. GB-A-2000805 describes a method for detecting bacteria in a medium by using an "electron transporter" which is capable of forming a redox system and of intervening in growth of cells so that the proportions of its oxidised and reduced form are modified. The oxido-reduction balance is then measured.

According to one aspect of the present invention there is provided a method for detecting anaerobic growth of a micro-organism in a sterile nutrient medium comprising the steps of:

a) forming a solution of said nutrient medium with an indicator species; and

b) sensing the change of an electrical property of said solution while in direct contact with two electrodes, one of which is a cathode,

characterised in that the electrical property is the potential difference developed galvanically between said two eectrodes, and

in that the indicator species is electrochemically reducible at the cathode to enable a current to flow through the solution and is consumed by the micro-organisms for their growth, the growth of the micro-organisms accordingly causing a change in said potential difference.

According to another aspect there is provided a method for detecting anaerobic growth of a micro-organism in a sterile nutrient medium comprising the stpes of:

a) forming a solution of said nutrient medium with an indicator species; and

b) sensing the change of an electrical property of said solution while in direct contact with two electrodes, one of which is a cathode,

characterised in that the electrical property is voltage or current developed between the two electrodes while an electrical DC input is applied across the two electrodes to maintain constant current or constant potential conditions respectively, and

in that the indicator species is electrochemically reducible at the cathode to enable a current to flow through the solution and is consumed by the micro-organisms for their growth, the growth of the micro-organism accordingly causing a change in said developed voltage or developed current.

The term "consumed" is used herein to denote a detectable alteration in the indicator species such that it can no longer be reduced at the relevant electrode.

A presently preferred indicator species is riboflavin. This has been found by the inventors to give start potentials which are comparable with existing aerobic detection systems and which remain steady until growth of micro-organisms reaches a particular level.

2

Using an aluminium (or aluminium alloy) anode the method preferably involves the addition to the nutrient medium of a phosphate buffer. This controls the pH of the medium and the dissolution of the aluminium of the anode, and so improves the signal-to-noise ratio of the system. Inorganic orthophosphates are suitable as the phosphate buffer.

From a second aspect thereof the invention also provides an apparatus for detecting anaerobic growth of a micro-organism. Accordingly such an apparatus comprises a nutrient medium in which has been dissolved an electrochemically reducible indicator species which is consumed by the micro-organism during anaerobic growth thereof, and, in contact with the nutrient medium, two electrodes with means for sensing, via said electrodes, a change in an electrical property of the nutrient medium during said anaerobic growth.

For a better understanding of the present invention and to show how the same may be carried into effect, reference will now be made by way of example to the accompanying drawings in which:-

Figure 1 is a diagrammatic sketch of an electrochemical detection apparatus; and

Figure 2 is a graph comparing aerobic and anaerobic detection systems.

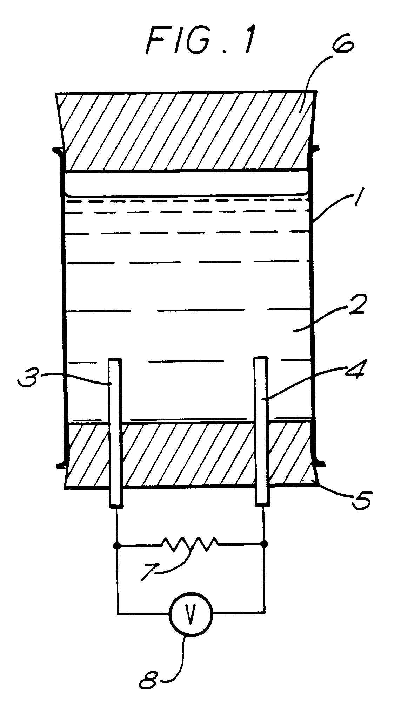

Figure 1 shows a detection system formed by an electrochemical cell comprising a container 1 in which is placed a sterile nutrient broth 2. A gold electrode 3 and an aluminium electrode 4 secured in a stopper 5 are placed in contact with the nutrient medium 2. A further stopper 6 seals the container 1. A resistor 7 is connected across the electrodes 3,4 with a voltmeter 8 for sensing the potential galvanically developed across the resistor. The operation of the detection system of Figure 1 for aerobic samples is known (for example, from our British Patent Specification No 2142433), and will not be described herein. However, to aid in understanding the present invention, an explanation of the mechanism underlying detection in aerobic samples will first be given.

The difference in absolute potentials between gold and aluminium electrodes in this system is of the order of 500mV. Current generated by this electropotential is passed through the resistance 7. The current arises from electrons flowing from the aluminium electrode to the gold electrode since the aluminium electrode has a more negative potential. The present inventors have reasoned that, if when electrons arrive at the gold they are not removed, then the potential of the gold would quickly become more negative and ultimately reach that of the aluminium. The current would then cease. In the presence of dissolved molecular oxygen, however, this does not occur but instead oxygen molecules are reduced at the gold electrode by accepting electrons therefrom. The source of electrons is maintained at the aluminium electrode by its slow dissolution as trivalently charged ions.

The reactions are probably

At the Al electrode :     $Al \longrightarrow Al^{3+} + 3e\text{-}$

At the Au electrode :     $O_2 + 2H_2O + 2e\text{-} \rightarrow H_2O_2 + 2OH^-$ and/or $O_2 + 2H_2O + 4e\text{-} \rightarrow 4OH$

In the absence of micro-organisms the oxygen in the medium 2 will result in a relatively large potential difference of about 400-500mV, which may slowly decline as some of this oxygen is consumed at the cathode. However, as soon as organisms are introduced and begin to grow they start to consume the oxygen, the content of which in the medium is therefore reduced at an accelerated rate. Eventually the oxygen is substantially consumed, the potential difference between the two electrodes approaches zero, and current flow declines correspondingly. The potential difference across the electrodes is measured by the voltmeter 8, which accordingly provides a method of detecting the micro-organisms.

As the mechanism relies on the consumption of free oxygen, it is not suitable for anaerobic micro-organisms. By applying their discovery of the aerobic mechanism to anaerobic systems the present inventors have identified the necessary qualities of an indicator species which could be used in place of oxygen for an anaerobic system. The criteria which the Applicants believe must be satisfied are

(1) that the indicator species should be electrochemically reducible;

(2) that it should be soluble in the nutrient medium; and

(3) that it can be utilised by the micro-organisms for growth. It is also desirable that the indicator species should withstand autoclaving to sterilise the nutrient medium. This is not essential since other sterilising procedures, e.g. filtering, could be adopted. There is no obvious route to the isolation of suitable species satisfying these criteria, but the inventors have discovered that riboflavin is suitable, and is preferred.

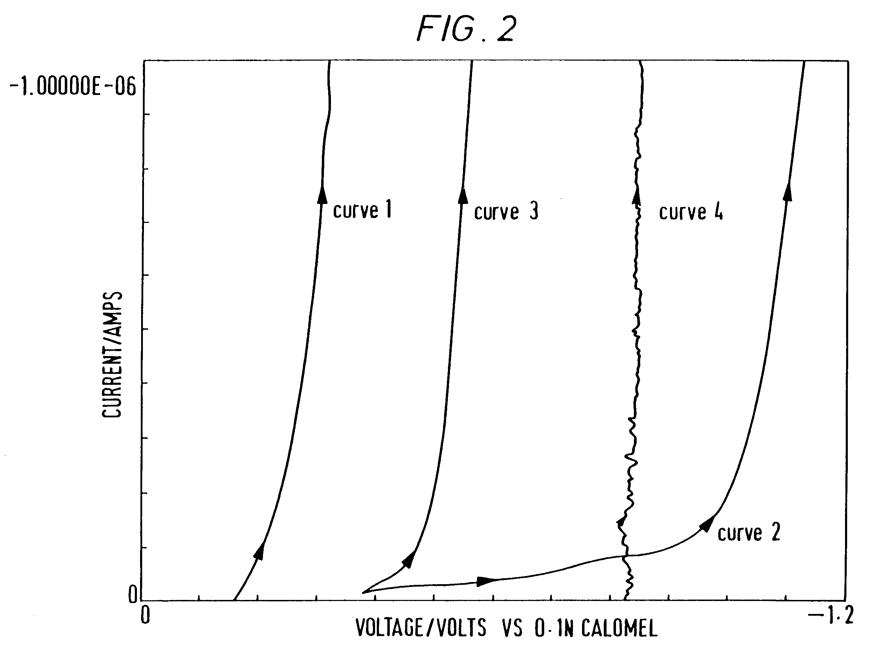

Figure 2 compares the use of riboflavin in an anaerobic system with an aerobic system (in which oxygen is the indicator species).

Figure 2, curve 1, shows the current/voltage curve for a fully aerated solution. The curve results from the reduction of oxygen and this is demonstrated by curve 2 which was obtained after de-oxygenating the same solution by bubbling nitrogen through it for about 2 hours. It is clear that under these conditions no current flows until much more negative potentials are reached, when the reduction of water occurs:-

$$2H_2O + 2e^- \longrightarrow H_2 + 2OH^-$$

However, when riboflavin is added to this anaerobic system, curve 3 is obtained indicating that the riboflavin is being electrochemically reduced.

Curve 4 shows the current/voltage curve for the dissolution of the aluminium electrode. (The modulus of the current has been taken since it has a positive sign because it is an oxidation reaction).

It is clear from these curves that the riboflavin behaves in a similar manner to oxygen and so should work in a detection system. However, because the curve for the riboflavin is closer to the aluminium curve than is the oxygen curve, a lower voltage would be expected when riboflavin is used in place of oxygen. It should also be noted that these experiments have been performed in salt solution, and some differences are to be expected when a micro-biological growth medium is used.

The Applicants have introduced riboflavin into anaerobic control media, and have recorded steady voltages of about 350-400mV. Moreover, when micro-organisms which have been introduced into the cells have grown, a significant reduction of this voltage to about 200mV has occurred.

While the above description relates to an Al/Au electrode cell, it will be appreciated that similar results could be obtained in an impressed voltage or current system as is described in European Patent Application Publication No EP-A-0313411.

Further experiments have been carried out to isolate species other than riboflavin which are suitable for use in an anaerobic detection system.

An initial experiment involved screening 14 compounds that either had similar chemical structures to riboflavin or had structures which could accept electrons. Since the majority of the compounds were insoluble in aqueous solutions and the effect of autoclaving was not considered, the following experimental protocol was used.

A control medium including the following constituents was prepared, using distilled water as solvent. The quantities given are per litre of distilled water.

CONTROL MEDIUM

| | |
|---|---|
| Proteose Peptone No 3 | 10g |
| Tryptone | 5g |
| Yeast Extract | 5g |
| D-Glucose | 2.5g |
| Sodium Chloride | 2.3g |
| Di-ammonium hydrogen orthophosphate | 7.3g |
| Glutathione | 4g |

After preparation the control medium was dispensed into containers and autoclaved for 15 minutes at 15 psi. Immediately after autoclaving the containers were stoppered.

1% w/v solutions/suspensions of the compounds to be tested were prepared in distilled water and 0.5ml added to 50ml of the control medium. The final concentration of each compound was a nominal 0.01%. Containers were then entered into an electrochemical detection system as follows, the experiments designated Lab Code 67-4 to 67-49 relating to the control medium with the addition of the compounds to be tested.

4

| LAB CODE | COMPOUND |
|---|---|
| 67-1 to 67-3 | Control, No Additions |
| 67-4 to 67-6 | Riboflavin (0.006%) |
| 67-7 to 67-9 | Nitrobenzene |
| 67-10 to 67-12 | 4-Nitrobenzaldehyde |
| 67-13 to 67-15 | 2-Nitroethanol |
| 67-16 to 67-18 | Nitrobenzoic Acid |
| 67-19 to 67-21 | p-Nitrophenylacetate |
| 67-22 to 67-24 | Folic Acid |
| 67-25 to 67-27 | 3-Nitro-L-Tyrosine |
| 67-28 to 67-30 | 2-Pyrazine Carboxylic Acid |
| 67-31 to 67-33 | 2,3 Dimethoxy-5-methyl 1,4 Benzoquinone |
| 67-34 to 67-36 | Menadione |
| 67-37 to 67-39 | Lumichrome |
| 67-40 to 67-42 | Lumazine Monohydrate |
| 67-43 to 67-45 | Nicotinamide |
| 67-46 to 67-49 | P-Benzoquinone |

The Applicants found that the control medium without any additions gave an unacceptably low signal. Moreover, the majority of the compounds listed above did not improve the signal level. However, two compounds, 4-Nitrobenzaldehyde and Lumichrome, when added to the control medium, resulted in a signal which was comparable to that given by the control medium with riboflavin. Therefore, Lumichrome, 4-Nitrobenzaldehyde and Naphthoquinone (which was not available for the initial screen but had previously been found to be effective) were examined further.

The same control medium as before was prepared, and the three compounds being studied further were added to amounts of this medium. The media produced were then sterilized either by autoclaving at 15 psi for 15 minutes or by filtration through a 0.22 $\mu$m membrane (with no precautions taken to keep the medium anaerobic). Immediately after sterilization the containers were stoppered and stored at room temperature for 24 hours. The containers were then entered into an electrochemical detection system as follows:-

| LAB CODE | ADDITION |
|---|---|
| 69A and 69AF | Riboflavin (0.006%) |
| 69B and 69BF | Naphthoquinone (0.0015%) |
| 69C and 69CF | 4 Nitrobenzaldehyde (0.006%) |
| 69D and 69DF | Lumichrome (0.002%) |
| (F denotes that the medium was filter sterilized). | |

It is to be noted that the solubility of the Napthoquinone and Lumichrome was such that even with the small amounts used, some of the compound failed to dissolve.

The containers were inoculated with either Clostridium tetani or Bacteroides fragilis on day 7 or Clostridium perfringens on day 8. Approximately 100 organisms were added to each bottle.

Initial "humps" appeared in the filter sterilized containers, probably the result of the presence of residual oxygen in the medium.

RIBOFLAVIN. The control containers gave the expected smooth signal of 400mV and the growth of the test organisms was indicated by a voltage drop of 150mV. Filter sterilized medium resulted in a signal of about 375mV and Cl. perfringens failed to grow and signal. Later experiments showed that some riboflavin derivatives, e.g. riboflavin phosphate, could be used instead of riboflavin.

NAPHTHOQUINONE. The voltage declined from 400mV to 300mV over the 10 day incubation period. Although growth of the three organisms was observed in this medium, the voltage drop was only 50mV. In contrast medium that had been filter sterilized gave a voltage output of 500mV. The signal from two of the bottles deteriorated after about day 5. Growth of B. fragilis was signalled by a voltage drop of 150mV whilst Cl. tetani gave a 200 - 250mV drop. Cl. perfringens failed to grow and signal. A later experiment showed that 2-hydroxy-1, 4-naphthoquinone at 0.004% concentration could be used as the indicator species.

5

NITROBENZALDEHYDE. The voltage output from autoclaved medium was approximately 325mV declining to 225mV over the incubation period. Although growth of the test organisms was observed there was no voltage change. The signal response from filter sterilized medium was variable. All three test organisms grew with B. fragilis signalling after 24 hours and Cl. tetani after 48 hours. Cl. perfringens failed to signal, although the voltage output from this container was some 100mV less than the other containers in the series.

LUMICHROME. The voltage output from autoclaved medium was less than 300mV and the growth of the test organisms was not signalled by a voltage drop. Filter sterilized medium gave a high voltage output (c 500mV) which declined over the ten day period. Cl. tetani failed to grow in this medium, whilst B. fragilis and a contaminant grew and signalled. (Cl. perfringens was not added to this series). The control container gave a response indicative of growth, but no contamination was visually observed.

In conclusion, filter sterilized media gave higher voltage outputs than media that had been autoclaved, suggesting that during autoclaving the compounds are either lost from the medium or changed in some way. However the signal generated by filtered media was generally less stable possible because some residual oxygen was present. It is believed that this problem could be overcome by filter sterilizing and dispensing hot media in an anaerobic atmosphere, increasing the amount of reducing agent (e.g. glutathione) and/or increasing the equilibration time.

The present inventors have examined the effect of varying the phosphate buffer concentration in the control medium, by monitoring the signal generated by the control medium with 0.006% riboflavin. Reduction of the phosphate buffer concentration from 200mM to 75mM was shown to have little effect on signal quality, although the time taken to reach maximum voltage increased from 3 hours (200mM buffer) to 12 hours (75mM buffer). However, the experiments were performed in the absence of human blood. In the experiments set out below modified control media were prepared with five different potassium phosphate buffer concentrations, and 5ml of human blood was added to the medial on entry into an electrochemical detection system.

Control medium containing 125mM potassium phosphate buffer and riboflavin at 0.006% concentration was diluted with the same control medium but lacking any phosphate buffer to give a series of test media containing 0, 25, 50, 75, 100 and 125mM buffer concentrations with 0.006% riboflavin. Regardless of buffer concentration, all the test media containing buffer had a pH of 7.3 after autoclaving whilst the corresponding pH value of the test medium without phosphate buffer was 7.8. Each test medium was dispensed into four containers, and the containers were autoclaved.

Human blood (5ml) was added to the containers immediately on cooling, prior to their entry into the detection system. Cl. tetani was inoculated into one container from each test medium.

All the inoculated containers showed a response, the voltage signal level increasing with the phosphate buffer concentration. However, high levels of buffer concentration were found to delay the response, it is believed because of inhibition of the microbial growth by the phosphate. Low levels of buffer concentration, on the other hand, gave low signal-to-noise ratios.

Further studies carried out by the Applicant have revealed or confirmed the following substances which can be used in place of riboflavin as the electrochemically reducible indicator species.

BATCH

RIBOFLAVIN TYPE COMPOUNDS

Lumichrome                                                    90


QUINONE TYPE COMPOUNDS

Anthraflavic Acid                                            93
Anthraquinone-2,5-sulphonic Acid                             92
Alizarin Red                                                 92
(6 or 7 or 8 or 9)-Chloro-1,3-dimethylbenzo(G)
  -pteridine-2,4-(1H,3H)-dione                               92
Fast Red Salt                                                92
2-Hydroxy-1,4-Naphthoquinone                                 90
1,4-Naphthoquinone                                           94
Purpurin                                                     94

## NITRO- TYPE COMPOUNDS

| | |
|---|---|
| 4-Nitrobenzyl Alcohol | 92,93 |
| 4-Nitrobenzaldehyde | 93 |
| m-Nitrobenzamidine | 93 |
| 4-Nitrobenzoyl-B-alanine | 94 |
| N(4-Nitrobenzoyl)-L-glutamic Acid | 92 |
| Nitrocinammaldehyde | 93 |

## OTHERS

| | |
|---|---|
| Reactive Blue 2 | 94 |
| Safranine | 94 |

The following compounds gave a signal that was marginally better than the control.

| | |
|---|---|
| P-Nitrophenyl Phosphate | 93 |

The following compounds gave a signal that was marginally better than the control and were not inoculated.

| | |
|---|---|
| 4-Nitrocatechol | 92 |
| 1,4-Diaminoanthraquinone | 92 |

The following compounds are also regarded as being potential alternatives to riboflavin.

Riboflavin Phosphate

Nitrobenzene

## Claims

1. A method for detecting anaerobic growth of a micro-organism in a sterile nutrient medium comprising the steps of:
   a) forming a solution of said nutrient medium with an indicator species; and
   b) sensing the change of an electrical property of said solution while in direct contact with at least one electrode,
   characterised in that step b) is carried out with the solution in direct contact with both an anode and a cathode electrically connected outside the medium in an electrical circuit
   in that the electrical property is related to current flowing in the electrical circuit as a result of reduction of the indicator species at the cathode, and

in that the indicator species comprises riboflavin or a derivative thereof which is reducible electrochemically at the cathode so as to remove electrons therefrom, the indicator species being furthermore consumed by the micro-organisms as they grow so that a predetermined level of growth results in a detectable change in said electrical property.

2. A method according to claim 1 wherein the anode is connected to the cathode outside the medium via a resistor and wherein the electrical property is the potential difference developed galvanically across said resistor.

3. A method according to claim 1 wherein the electrical circuit includes means for applying an electrical DC input between the anode and the cathode to maintain constant current or constant potential conditions and wherein the electrical property is voltage or current respectively.

4. A method as claimed in any preceding claim in which the electrochemically reducible indicator species comprises riboflavin at a concentration in the nutrient medium of about .006%.

5. A method as claimed in any of claims 1 to 3 wherein the indicator species comprises riboflavin phosphate.

6. A method as claimed in claim 1 or 2 in which the anode electrode comprises aluminium.

7. A method as claimed in claim 6 wherein a phosphate buffer is added to the nutrient medium.

8. A method as claimed in claim 7 wherein the phosphate buffer comprises potassium orthophosphate.

9. An apparatus for detecting anaerobic growth of a micro-organism in accordance with the method of any preceding claim, which comprises a sterile nutrient medium in which has been dissolved an electrochemically reducible indicator species comprising riboflavin or its derivatives which is consumed by the micro-organisms during anaerobic growth thereof and, in con = act with the nutrient medium, an anode and a cathode (3,4), the apparatus further comprising means (7,8) for sensing a change in an electrical property of the nutrient medium during said anaerobic growth, said electrical property being related to current flow as a result of reduction of the indicator species at the cathode.

10. An apparatus as claimed in claim 8 in which the cathode is gold and the anode comprises aluminium, the electrical property being detected as a change in galvanically generated potential across a resistor (7) connected between the anode and the cathode (3,4).

**Patentansprüche**

1. Verfahren zum Nachweisen des anaerobischen Wachstums eines Mikroorganismus in einem sterilen Nährmedium, wobei das Verfahren die folgenden Schritte umfaßt:
a) Herstellen einer Lösung des Nährmediums mit einer Indikatorsubstanz; und
b) Messer der Änderung einer elektrischen Eigenschaft der Lösung, während diese in direktem Kontakt mit wenigstens einer Elektrode steht,
dadurch gekennzeichnet, daß Schritt b) ausgeführt wird, während die Lösung in direktem Kontakt sowohl mit einer Anode als auch mit einer Kathode steht, die außerhalb des Nährmediums in einen elektrischen Schaltkreis eingeschaltet sind,
daß die elektrische Eigenschaft sich auf den Stromfluß in den elektrischen Schaltkreis, der infolge der Reduktion dar Indikatorsubstanz an der Kathode fließt, bezieht, und
daß die Indikatorsubstanz Riboflavin oder einen Abkömmling desselben enthält, wobei die Indikatorsubstanz an der Kathode elektrochemisch derart reduzierbar ist, daß Elektronen aus derselben entfernt werden, und wobei die Indikatorsubstanz ferner durch die Mikroorganismen während deren Wachstum derart verbraucht wird, daß eine vorbestimmte Wachstumsgröße zu einer nachweisbaren Änderung in der elektrischen Eigenschaft führt.

2. Verfahren nach Anspruch 1, bei welchem die Anode mit der Kathode außerhalb des Nährmediums über einen Widerstand verbunden ist und die elektrische Eigenschaft die galvanisch am Widerstand

9

EP 0 397 362 B1

entstehende Potentialdifferenz ist.

3. Verfahren nach Anspruch 1, bei welchem der elektrische Schaltkreis eine Anordnung zum Anlegen einer elektrischen Eingangsgleichspannung zwischen der Anode und der Kathode aufweist, um konstante Strom- oder konstante Potentialbedingungen aufrechtzuerhalten, und bei welchem die elektrische Eigenschaft Spannung bzw. Strom ist.

4. Verfahren nach einem der vorangehenden Ansprüche, bei welchem die elektrochemisch reduzierbare Indikatorsubstanz Riboflavin in einer Konzentration in dem Nährmedium von etwa 0,006% aufweist.

5. Verfahren nach einem der Ansprüche 1 bis 3, bei welchem die Indikatorsubstanz Riboflavinphosphat aufweist.

6. Verfahren nach Anspruch 1 oder 2, bei welchem die Anode aus Aluminium besteht oder Aluminium enthält.

7. Verfahren nach Anspruch 6, bei welchem ein Phosphatpuffer zu dem Nährmedium zugegeben wird.

8. Verfahren nach Anspruch 7, bei welchem der Phosphatpuffer Kaliumorthophosphat enthält.

9. Vorrichtung zum Nachweisen des anaerobischen Wachstums eines Mikroorganismus gemäß dem Verfahren nach einem der vorangehenden Ansprüche, welche aufweist: ein steriles Nährmedium, in welchem eine elektrochemisch reduzierbare Indikatorsubstanz gelöst ist, die Riboflavin oder deren Abkömmlinge enthält und welche durch die Mikroorganismen während des anaerobischen Wachstums derselben verbraucht wird, sowie in Kontakt mit dem Nährmedium eine Anode und eine Kathode (3,4), wobei die Vorrichtung ferner eine Anordnung (7,8) zum Messen einer Änderung einer elektrischen Eigenschaft der Nährsubstanz während des anaerobischen Wachstums aufweist und wobei die elektrische Eigenschaft auf den elektrischen Stromfluß bezogen ist, der sich aus der Reduktion der Indikatorsubstanz an der Kathode ergibt.

10. Vorrichtung nach Anspruch 9, bei welcher die Kathode aus Gold besteht und die Anode Aluminium aufweist, wobei die elektrische Eigenschaft als eine Änderung des galvanisch erzeugten Potentials an einem Widerstand (7) festgestellt wird, der zwischen die Anode und die Kathode (3,4) geschaltet ist.

**Revendications**

1. Procédé de détection de croissance anaérobie d'un micro-organisme dans un milieu nutritif stérile, comprenant les étapes consistant à :
   a) former une solution dudit milieu nutritif avec une espèce d'indicateur ; et
   b) détecter le changement d'une propriété électrique de ladite solution pendant que celle-ci est en contact direct avec au moins une électrode,
   caractérise en ce qu'on effectue l'étape b) pendant que la solution est en contact direct avec une anode et une cathode qui sont reliées électriquement à l'extérieur du milieu dans un circuit électrique,
   en ce que la propriété électrique est une fonction du courant passant dans le circuit électrique, comme le résultat d'une réduction de l'espèce d'indicateur à la cathode, et
   en ce que l'espèce d'indicateur est constituée de riboflavine ou d'un dérivé de celle-ci, qui peut être réduite électrochimiquement à la cathode de manière à en chasser les électrons, l'espèce d'indicateur étant consommée, en outre, par les micro-organismes au fur et à mesure que ceux-ci se développent, de sorte qu'un taux prédéfini de croissance conduit à un changement décelable de ladite propriété électrique.

2. Procédé selon la revendication 1, dans lequel l'anode est reliée à la cathode, à l'extérieur du milieu, par l'intermédiaire d'une résistance, et dans lequel la propriété électrique est la différence de potentiel développée de façon galvanique de part et d'autre de ladite résistance.

3. Procédé selon la revendication 1, dans lequel ledit circuit électrique comprend un moyen pour appliquer une entrée de courant électrique continu entre l'anode et la cathode pour maintenir des conditions constantes de courant ou des conditions constantes de potentiel, et dans lequel la propriété électrique est respectivement la tension ou le courant.

4.  Procédé tel que revendiqué dans l'une quelconque des revendications précédentes, dans lequel l'espèce d'indicateur pouvant être réduite électrochimiquement est constituée de riboflavine a une concentration d'environ 0,006 % dans le milieu nutritif.

5.  Procédé tel que revendiqué dans l'une quelconque des revendications 1 à 3, dans lequel l'espèce d'indicateur est constituée de phosphate de riboflavine.

6.  Procédé tel que revendiqué dans la revendication 1 ou 2, dans lequel l'électrode formant anode est constituée d'aluminium.

7.  Procédé tel que revendiqué dans la revendication 6, dans lequel on ajoute un tampon de phosphate au milieu nutritif.

8.  Procédé tel que revendiqué dans la revendication 7, dans lequel le tampon de phosphate est constitué d'orthophosphate de potassium.

9.  Appareil de détection de croissance anaérobie d'un micro-organisme selon le procédé de l'une quelconque des revendications précédentes, comprenant un milieu nutritif stérile dans lequel on a dissous une espèce d'indicateur pouvant être réduite électrochimiquement, constituée de riboflavine au de ses dérivés, qui est consommée par le micro-organisme pendant la croissance anaérobie de celui-ci, ainsi qu'une anode et une cathode (3, 4) au contact du milieu nutritif, l'appareil comprenant, en outre, un moyen (7, 8) pour détecter un changement d'une propriété électrique du milieu nutritif pendant ladite croissance anaérobie, ladite propriété électrique étant une fonction du flux de courant, comme le résultat d'une réduction de l'espèce d'indicateur à la cathode.

10. Appareil tel que revendiqué dans la revendication 9, dans lequel la cathode est en or, et l'anode est constituée d'aluminium, la propriété électrique étant détectée sous la forme d'un changement du potentiel, qui est produit de façon galvanique, de part et d'autre d'une résistance (7) reliée entre l'anode et la cathode (3, 4).

FIG . 1

FIG. 2